# EUROPEAN PATENT SPECIFICATION

(11) **EP 2 641 545 B1**
(45) Date of publication and mention of the grant of the patent: **17.06.2020**
(21) Application number: 11840236.1
(22) Date of filing: 14.11.2011
(51) Int. Cl.: A61B 10/02, G01N 1/10

(54) **SWAB**
TUPFER
ÉCOUVILLON

(30) Priority: 12.11.2010 JP 2010253942
(43) Date of publication of application: 25.09.2013
(73) Proprietor: Nipro Corporation, Osaka-shi, Osaka 531-8510 (JP)
(72) Inventor: HONDA, Minoru, Osaka-shi Osaka 531-8510 (JP); TOMITA, Yoshikatsu, Osaka-shi Osaka 531-8510 (JP); ITO, Yoshiaki, Marugame-shi Kagawa 763-8605 (JP); KUWANO, Tomomasa, Marugame-shi Kagawa 763-8605 (JP); TOYA, Tsutomu, Marugame-shi Kagawa 763-8605 (JP)
(74) Representative: Bertsch, Florian Oliver
(86) International application number: PCT/JP2011/076169
(87) International publication number: WO 2012/063956

(56) References cited:
- EP-A1- 0 357 892
- EP-A1- 0 402 140
- EP-A1- 1 234 543
- WO-A1-81/00356
- FR-A5- 2 076 769
- JP-A- H09 188 777
- JP-A- 2006 104 436
- JP-A- 2007 139 556
- JP-U- S6 110 169
- JP-U- 61 010 169
- US-A- 3 228 398
- US-A- 5 475 890
- US-A1- 2005 014 858
- US-A1- 2008 119 579

## Description

### TECHNICAL FIELD

The present invention relates to swabs including a handle portion and a spherical tip portion, and particularly to a swab for specimen collection for testing for an infection.

### BACKGROUND ART

Generally, a cotton-tipped stick is used for testing for an infection. The cotton-tipped stick includes a handle portion made of paper or resin such as polyethylene, and a wad of cotton provided at the tip of the handle portion. The handle portion serves as a gripping portion for gripping the cotton-tipped stick, and the wad of cotton serves as a specimen collecting portion for collecting a specimen.

During actual specimen collection, a widely employed method is to insert the cotton-tipped stick into a patient's nasal cavity or oral cavity and rub the specimen collecting portion against the pharynx.

The specimen thus collected is extracted into extraction liquid which is then dripped onto a reaction plate, to determine the presence or absence of infection based on the color tone of an indicator line that appears on the reaction plate after a certain period of time (e.g., approximately several tens of minutes).

Japanese Patent Publication No. 04-31699 (Patent Document 1) describes employing a discontinuous pore structure at the tip of a swab.

### CITATION LIST

### PATENT DOCUMENT

PTD 1: Japanese Patent Publication No. 04-31699

### SUMMARY OF INVENTION

### TECHNICAL PROBLEM

If the specimen collecting portion has insufficient water absorbency, the amount of specimen dripped onto the reaction plate may be too small or may vary during the above testing. In this regard, the swab described in Patent Document 1 has a discontinuous pore structure at its tip, where the pores do not contribute to the improvement in water absorbency because they are discontinuous.

Furthermore, since the specimen collecting portion formed of a wad of cotton is hard and does not always have sufficient water absorbency, the patient may feel pain during specimen collection. A specimen collecting portion formed by electrostatic flocking is soft and suppresses pain to the patient. The specimen collecting portion by electrostatic flocking, however, tends to collect not only a desired specimen but a solid material such as dried mucus, and this solid material is caught in a filter and the like in a container of the extraction liquid, resulting in difficulty in performing the extraction operation.

The present invention was made in view of the problems as described above, and an object of the present invention is to provide a swab including a specimen collecting portion of high water absorbency and also offering excellent usability.

Other relevant documents include US 3 228 398 A which discloses a swab including a flexible handle and a sponge with a spherical tip made of partially open celled polyurethane foam. US 2008/119579 A1 which discloses wet type polyurethane foam suitable for swabs and JP H09 188777 A which discloses wet-type polyurethane foam with a 80-200µm cell diameter, suitable for a "make-up tip applicator" or sensitive sites in the skin.

### SOLUTION TO PROBLEM

A swab according to the present invention is a swab for specimen collection, which includes a flexible handle portion, and a spherical tip portion provided at a tip portion of the handle portion and made of wet-type urethane sponge having a membrane structure of continuous pores.

In the wet-type urethane sponge, the space left after a pore-producing agent has been eluted is referred to as pores. Polyurethane resin including the "pores" is referred to as a polyurethane porous body. The "pores" are classified into "discontinuous pores" in which the pores are discontinuous with and isolated from one another, and "continuous pores" in which the pores are continuous with one another.

The "continuous pores" as used herein are meant to refer to a structure in which the pores are continuous with one another in the wet-type urethane sponge which is a porous body. In contrast, the "discontinuous pores" are meant to refer to a structure in which the pores are discontinuous with and isolated from one another.

Furthermore, the "continuous pores" as used herein are classified into a "membrane structure" and a "mesh structure." The "membrane structure" is meant to refer to a structure in which the pores are uniformly distributed in a three-dimensional direction and continuous with one another, and the surfaces of the pores (namely, the borders between adjacent pores) are formed of a resin film and an opening formed in the resin film. In contrast, the "mesh structure" is meant to refer to a structure in which the resin framework coupled in a three-dimensional direction is provided in the form of a mesh, and the pores formed between the framework portions are continuous with one another, without a resin film closing the mesh.

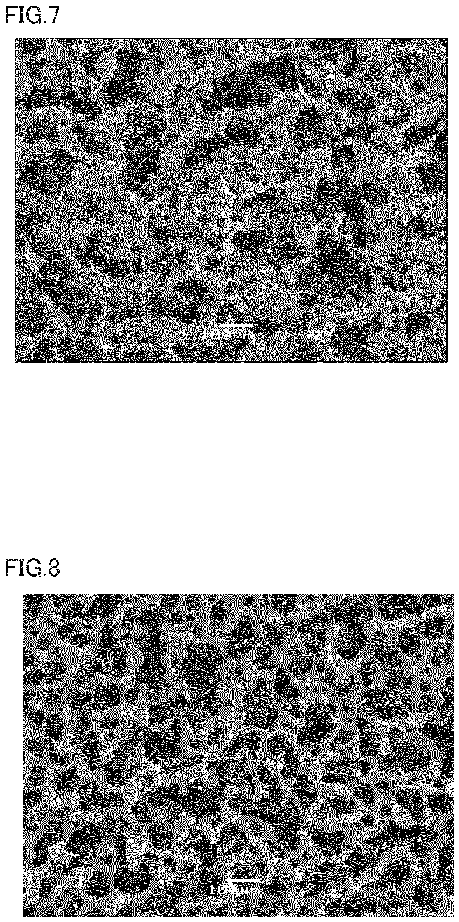

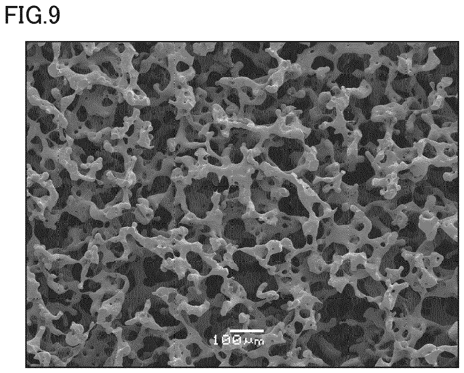

Fig. 7 shows an example of the "membrane structure" (SEM picture), and Figs. 8 and 9 show examples of the "mesh structure" (SEM pictures). As shown in Fig. 7, in the case of the "membrane structure," on a cut surface for SEM observation, a cut section of a resin film forming the borders between pores is observed and an opening formed in the resin film can be confirmed. On the other hand, as shown in Figs. 8 and 9, in the case of the "mesh structure," the resin film as was found in the "membrane structure" is not observed on a cut surface, but a state is seen where cut surfaces of a framework in a circular shape, a substantially circular shape, or an elliptical shape, a substantially elliptical shape are independently scattered.

In general, an "urethane sponge" is classified into a "wet-type urethane sponge" and a "dry-type urethane sponge." The "wet-type urethane sponge" as used herein is meant to refer to a porous body obtained by coagulating a mixture of urethane resin dissolved in a solvent such as dimethylformamide and an aqueous pore producing agent in coagulation liquid into a desired shape, and subsequently eluting the pore producing agent. In contrast, the "dry-type urethane sponge" is meant to refer to foam obtained by mixing a compound which is gasified by heat and the like, or by utilizing carbon dioxide gas which is generated by the reaction of isocyanate and water as a blowing agent, during a polymerization process for urethane resin. Due to such production method, it is usually said to be difficult to make the pore diameter of the dry-type urethane sponge smaller than 200 µm.

The above-described wet-type urethane sponge is obtained, for example, by the reaction of a polyol component containing a high-molecular-weight polyol and a chain elongation agent, and a polyisocyanate compound. The high-molecular-weight polyol may be polyether-based polyol such as polypropylene glycol, polytetramethylene glycol, and polymer polyol, adipate-based polyol, polyester-based polyol such as polycaprolactone polyol, polycarbonate polyol, polyolefin polyol, and the like, and has a desirable molecular weight of 500 to 10000. The chain elongation agent may be ethylene glycol, 1, 4-butanediol, 1, 6-hexanediol, 1,5-pentanediol, 3-methyl-1,5-pentanediol, 1,3-propanediol, and the like. The polyisocyanate compound may be aromatic-based isocyanate such as methylene diphenyl diisocyanate, tolylene diisocyanate, xylylene diisocyanate, naphthylene-1,5-diisocyanate, and tetramethylene xylylene diisocyanate, alicyclic-based isocyanate such as isophorone diisocyanate and dicyclohexyl methane diisocyanate, and aliphatic-based isocyanate such as hexamethylene diisocyanate, dimer acid diisocyanate, and norbornene diisocyanate, and the like.

It is preferable that the spherical tip portion have a hardness in the range from 20° to 80° (room temperature) as measured with the ASKER® Durometer Type F. A hardness of less than 20° results in too much softness and the resilience of the sponge is lost. A hardness of more than 80° results in too much hardness and may cause pain to the patient. The hardness set at from 20° to 80° allows for a smooth and pliable texture without the impact resilience becoming too high.

The hardness of the spherical tip portion made of the above wet-type urethane sponge can be arbitrarily controlled by the selection of a combination and a mixing ratio of the polyol component and the polyisocyanate compound for use in polyurethane synthesis.

In one embodiment, the spherical tip portion is formed by welding two pieces of sponge sandwiching the tip portion of the handle portion therebetween to the handle portion, and a base end portion of the spherical tip portion has a shape that decreases in diameter with increasing distance from the tip of the handle portion.

More preferably, the spherical tip portion has a substantially cylindrical shape with a spherically shaped tip. Further preferably, the spherical tip portion further includes a slim cylindrical portion formed continuously with the base end portion side with respect to a portion in the substantially cylindrical shape (cylindrical portion having a smaller diameter than the portion in the substantially cylindrical shape).

In one embodiment, the handle portion contains not less than 10 wt percent and not more than 30 wt percent of talc.

In one embodiment, the wet-type urethane sponge does not contain an antioxidant or an ultraviolet absorbing agent as an additive.

In one embodiment, the wet-type urethane sponge has a bubble point diameter of not less than 50 µm and not more than 150 µm. A bubble point diameter of less than 50 µm results in a reduced amount of absorption of a specimen, and a bubble point diameter of more than 150 µm causes the pores to be too coarse, also resulting in a reduced amount of absorption of a specimen. The bubble point diameter set at not less than 50 µm and not more than 150 µm can increase the amount of absorption of a specimen.

The bubble point diameter as used herein is meant to refer to a maximum pore diameter as measured with the method described in ASTM F316-03 and JIS K3832 (bubble point method). More specifically, Pore Size Meter PSM 165 (manufactured by YUASA-IONICIS COMPANY, LIMITED) is used as test equipment, and ethanol is used as a solvent.

### ADVANTAGEOUS EFFECTS OF INVENTION

According to the present invention, a swab for specimen collection for testing for an infection and for application of an antiseptic and the like, including a specimen collecting portion of high water absorbency and also offering excellent usability, can be provided.

### BRIEF DESCRIPTION OF DRAWINGS

Fig. 1 is a front view showing a swab according to one embodiment of the present invention.
Fig. 2 is an enlarged exploded view showing the structure of a tip portion of the swab shown in Fig. 1.
Fig. 3 is a graph showing comparison between water absorbency of swabs according to Examples 1 to 3 of the present invention and water absorbency of swabs according to Comparative Examples A1, A2, B1 and B2.
Fig. 4 is a graph illustrating a color tone of an indicator line for a first specimen, showing comparison between the color tone when the swab according to Example 1 of the present invention was used, and the color tones when the swabs according to Comparative Examples A1 and A2 were used.
Fig. 5 is a graph illustrating a color tone of an indicator line for a second specimen, showing comparison between the color tone when the swab according to Example 1 of the present invention was used, and the color tones when the swabs according to Comparative Examples A1 and A2 were used.
Fig. 6 shows a variation of the structure of the tip portion of the swab.
Fig. 7 is a SEM picture showing an example of a "membrane structure."
Fig. 8 is a SEM picture showing an example of a "mesh structure."
Fig. 9 is a SEM picture showing another example of the "mesh structure."

### DESCRIPTION OF EMBODIMENTS

The embodiments of the present invention will be described below. It is noted that the same or corresponding parts are designated by the same reference characters, and description thereof may not be repeated.

When any reference is made to a number, an amount and the like in the embodiments described below, the scope of the present invention is not necessarily limited to that number, amount and the like unless otherwise specified. Moreover, in the following embodiments, each constituent element is not necessarily a requirement of the present invention unless otherwise specified.

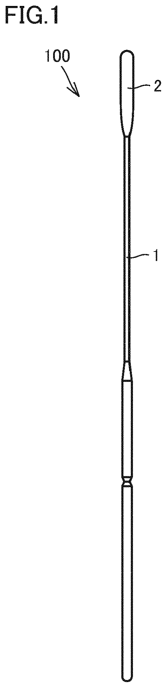

Fig. 1 is a front view showing a swab according to this embodiment. As shown in Fig. 1, a swab 100 includes a handle portion 1 and a spherical tip portion 2.

Handle portion 1 is a portion serving as a gripping portion. Handle portion 1 is flexible and made of resin such as polypropylene or polyethylene. Preferably, handle portion 1 contains approximately not less than 10 wt percent and not more than 30 wt percent of talc. Consequently, suitable flexibility can be obtained.

Spherical tip portion 2 is a portion to be inserted into a patient's nasal cavity or oral cavity to collect a specimen. Spherical tip portion 2 according to this embodiment is made of wet-type urethane sponge. Spherical tip portion 2 has a membrane structure of continuous pores. Consequently, spherical tip portion 2 has dramatically improved water absorbency. Moreover, by employing the wet-type urethane sponge for spherical tip portion 2 which comes in direct contact with the patient, pain to the patient is suppressed.

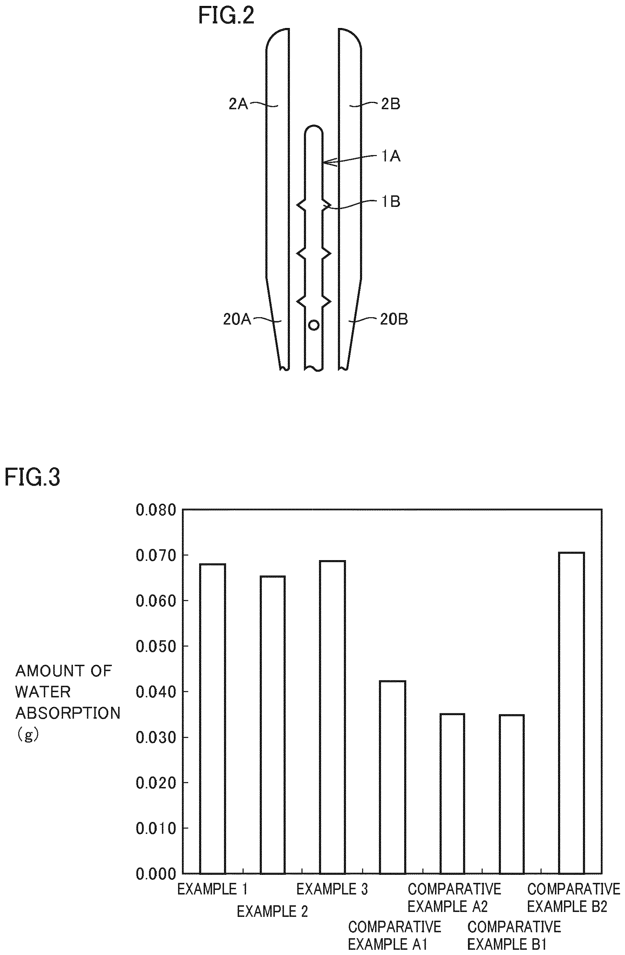

Fig. 2 is an enlarged exploded view showing the structure of a tip portion of swab 100. As shown in Fig. 2, a tip portion 1A of handle portion 1 is provided with projections 1B. Spherical tip portion 2 is formed of two pieces of sponge 2A and 2B. Pieces of sponge 2A and 2B are provided to sandwich tip portion 1A of handle portion 1 therebetween. Pieces of sponge 2A and 2B engage projections 1B of tip portion 1A. Consequently, spherical tip portion 2 is securely held to handle portion 1.

Pieces of sponge 2A and 2B may be joined to tip portion 1A by welding (e.g., heating for three seconds at 160°C) or by bonding. In a typical example, base end portions 20A and 20B of pieces of sponge 2A and 2B each has a shape that decreases in diameter with increasing distance from the tip of handle portion 1. Consequently, removal of pieces of sponge 2A and 2B from handle portion 1 can be prevented, thereby eliminating the need to fix pieces of sponge 2A and 2B by bonding.

Furthermore, in a typical example, pieces of sponge 2A and 2B do not contain an antioxidant or an ultraviolet absorbing agent as an additive. Consequently, an adverse effect (cytotoxic effect) on cells in the human body can be further reduced.

As described above, swab 100 according to this embodiment is characterized in that spherical tip portion 2 is made of wet-type urethane sponge having a membrane structure of continuous pores.

Fig. 3 is a graph showing comparison between water absorbency of swabs according to examples of the present invention and water absorbency of swabs according to comparative examples.

It is noted that the swabs in Examples 1 to 3 shown in Fig. 3 are swabs including a specimen collecting portion made of wet-type urethane sponge (TECHNOPOROUS®, product item: 451-W2 (planned)) manufactured by FUSHIMI Pharmaceutical Co., Ltd. On the other hand, the swabs in Comparative Examples A1 and A2 shown in Fig. 3 are commercially available swabs including a specimen collecting portion made of cotton, and the swabs in Comparative Examples B1 and B2 are swabs including a specimen collecting portion formed by electrostatic flocking.

Water absorbency tests were conducted with the following method. First, the dry weight of each swab was measured. Subsequently, tubes (BIO-BIK®) each containing 0.5 mL of "Purorasuto Flu specimen extraction liquid" were prepared in a number corresponding to the number of swabs. After each swab was dipped in the "Purorasuto Flu specimen extraction liquid" in the BIO-BIK tube for ten seconds, the weight of the swab was measured, and the dry amount was subtracted from the weight after the liquid absorption to obtain an amount of liquid absorption.

As shown in Fig. 3, the water absorbency is markedly improved in Examples 1 to 3 of the present invention relative to that of the commercially available swabs including a specimen collecting portion made of cotton (Comparative Examples A1 and A2). Consequently, the patient's specimen can be efficiently collected.

As shown in Fig. 3, on the other hand, comparing the water absorbency in Examples 1 to 3 of the present invention with the water absorbency of the swabs including a specimen collecting portion formed by electrostatic flocking (Comparative Examples B1 and B2), although the water absorbency in Comparative Example B1 is low, the water absorbency in Comparative Example B2 is equal to those in Examples 1 to 3.

However, the specimen collecting portion formed by electrostatic flocking as in Comparative Examples B1 and B2 tends to collect a solid material such as dried mucus, which tends to be caught in a filter and the like in a container of the extraction liquid, resulting in difficulty in performing the extraction operation.

The swabs according to Examples 1 to 3 of the present invention are highly advantageous in terms of water absorbency relative to the swabs including a specimen collecting portion made of cotton (Comparative Examples A1 and A2), and are highly advantageous in that an unnecessary solid material is not collected relative to the swabs including a specimen collecting portion formed by electrostatic flocking (Comparative Examples B1 and B2).

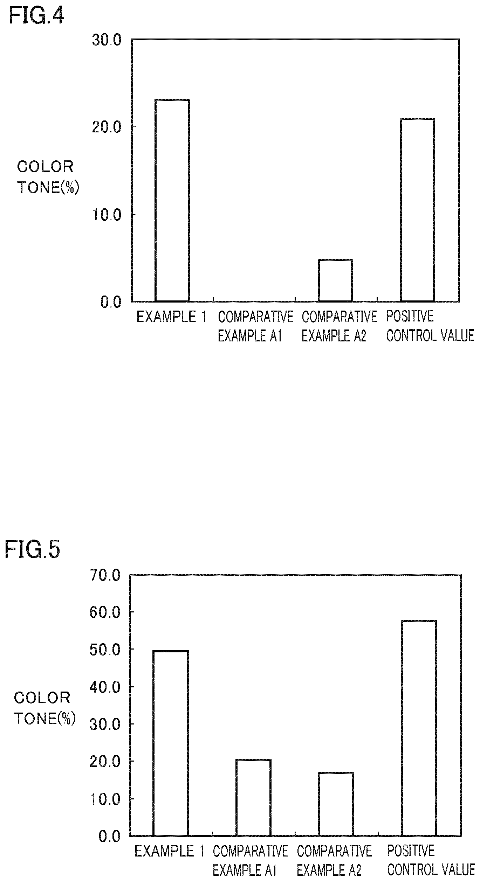

Next, the results of comparison (recovery test) between the color tone of an indicator line in Example 1 and the color tones of an indicator line in Comparative Examples A1 and A2 where the specimen collecting portion was made of cotton are described with reference to Figs. 4 and 5.

It is noted that Figs. 4 and 5 show comparison results of a A positive control and a B positive control of a BD Flu Examan® influenza virus kit (for detection of type A antigen and type B antigen), respectively.

For Example 1 and Comparative Examples A1 and A2, tests were conducted with the following method to observe the color tones of the indicator line. First, separate BIO-BIK tubes containing 0.5 mL of the A positive control and 0.5 mL of the B positive control, respectively, were prepared. Each swab was dipped in the positive controls in the tubes for ten seconds, and the swab that has been dipped in the positive controls was soaked in a tube containing 0.8 mL of extraction liquid and stirred, before being removed while the portion of a wad of cotton was squeezed. For each swab, five tubes of this extraction liquid were prepared for each of A and B. Each sample solution was tested with a test card (ImunoAce Flu manufactured by TAUNS Laboratories, Inc.), and a determination was made after fifteen minutes with an image analysis system (Densitograph manufactured by ATTO Corporation).

As is clear from the results shown in Figs. 4 and 5, the color tone of the indicator line is markedly improved in Example 1 relative to those in Comparative Examples A1 and A2. That is, in Example 1, owing to its excellent water absorbency relative to those in Comparative Examples A1 and A2, the specimen can be efficiently collected accordingly, leading to the markedly improved color tone.

It is noted that a "positive control value" in Figs. 4 and 5 represents a color tone change in the indicator line when a certain amount of specimen (0.06 g in this case) dissolved in specimen extraction liquid was dripped onto a reaction plate.

These results also demonstrate that, even considering the difference in the respective absorption amounts, the specimen can be readily released from the specimen collecting portion to a developer in the example as compared to the comparative examples. It can be seen that the swab in the example is suitable particularly for specimen collection.

The present inventors made a comparison between common cotton-tipped sticks and sponge swabs in greater detail. The results are shown in Tables 1 and 2.

It is noted that in Tables 1 and 2, a "common cotton-tipped stick" is meant to refer to a traditional cotton-tipped stick including a specimen collecting portion made of cotton, a "sponge swab" is meant to refer to forming a specimen collecting portion of "wet-type urethane sponge" in the examples of the present invention, and "dry-type urethane" is meant to refer to forming a specimen collecting portion of "dry-type urethane sponge."

**[Table 1]**

| Water absorbency test | | | |
|---|---|---|---|
| | Amount of water absorption (g) | Structure | Bubble point diameter (µm) |
| Common cotton-tipped stick 1 | 0.0422 | - | - |
| Common cotton-tipped stick 2 | 0.0351 | - | - |
| Sponge swab 1 | 0.0685 | Membrane structure | 81 |
| Sponge swab 2 | 0.0587 | Membrane structure | 62 |
| Sponge swab 3 | 0.0586 | Membrane structure | 113 |
| Sponge swab 4 | 0.0314 | Mesh structure | 66 |
| Sponge swab 5 | 0.0283 | Mesh structure | 136 |
| Dry-type urethane | 0.0400 | - | - |

**[Table 2]**

| Recovery test | | | |
|---|---|---|---|
| | Brightness | Structure | Bubble point diameter (µm) |
| Common cotton-tipped stick 1 | 22 | - | - |
| Common cotton-tipped stick 2 | 19 | - | - |
| Sponge swab 1 | 68 | Membrane structure | 81 |
| Sponge swab 2 | 76 | Membrane structure | 62 |
| Sponge swab 3 | 73 | Membrane structure | 113 |
| Sponge swab 4 | 34 | Mesh structure | 66 |
| Sponge swab 5 | 37 | Mesh structure | 136 |
| Dry-type urethane | 41 | - | - |

As shown in Tables 1 and 2, by employing the membrane structure rather than the mesh structure, even the sponge swabs of continuous pores can have a substantially increased water absorption amount and recovery amount. It is noted that "brightness" in Table 2 is proportional to virus concentration collected.

While the bubble points of the urethane sponges can be changed as appropriate, they are preferably not less than 50 µm and not more than 150 µm based on the results in Tables 1 and 2.

In addition to being applied to collect a specimen, the swab according to this embodiment can be used, for example, to apply an antiseptic and the like.

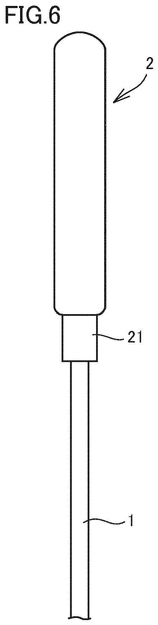

Fig. 6 shows a variation of the structure of the tip portion of the swab. As shown in Fig. 6, spherical tip portion 2 has a substantially cylindrical shape with a spherically shaped tip. Spherical tip portion 2 further includes a slim cylindrical portion 21 formed continuously with the base end portion side with respect to the portion in the substantially cylindrical shape.

The swab according to this embodiment has a shape which is not partially bulged like a common cotton-tipped stick, and can therefore lessen fear of the patient and reduce invasion. Moreover, when the swab is removed from a nasal cavity, the shape of the base end portion can lower the possibility of the swab being caught (the feeling that it will be caught) due to the small entrance of the nasal cavity.

Although the embodiments of the present invention have been described above, it should be understood that the embodiments disclosed herein are illustrative and non-restrictive in every respect. The scope of the present invention is defined by the terms of the claims, and is intended to include any modifications within the scope and meaning equivalent to the terms of the claims.

### INDUSTRIAL APPLICABILITY

The present invention is applicable to a swab for specimen collection for testing for an infection and the like.

### REFERENCE SIGNS LIST

1 handle portion; 1A tip portion; 1B projection; 2 spherical tip portion; 2A, 2B piece of sponge; 20A, 20B base end portion; 21 slim cylindrical portion; 100 swab.

## Claims

1. A swab comprising:
a flexible handle portion (1); and
a spherical tip portion (2) provided at a tip portion of said handle portion (1) and made of wet-type urethane sponge having a structure in which pores are uniformly distributed in a three-dimensional direction and continuous with one another, and the borders between adjacent pores are formed of a resin film and an opening formed in said resin film, and in which urethane resin dissolved in a solvent has been coagulated in coagulation liquid,
said wet-type urethane sponge having a maximum pore diameter, which is measured with the bubble point method described in JIS K3832-1990, of not less than 62 µm and not more than 113 µm.

2. The swab according to claim 1, wherein
said spherical tip portion (2) is formed by welding two pieces of sponge (2A, 2B) sandwiching said tip portion of said handle portion (1) therebetween to said handle portion (1), and
a base end portion of said spherical tip portion (2) has a shape that decreases in diameter with increasing distance from said tip portion of said handle portion (1).

3. The swab according to claim 1 or 2, wherein
said spherical tip portion (2) has a substantially cylindrical shape with a spherically shaped tip.

4. The swab according to claim 3, wherein
said spherical tip portion (2) further includes a slim cylindrical portion (21) formed continuously with a base end portion side.

## Patentansprüche

1. Tupfer, umfassend:
einen flexiblen Griffbereich (1) und
einen sphärischen Spitzenbereich (2), der am Spitzenbereich des Griffbereichs (1) angeordnet ist und aus Feucht-Typ-Urethan-Schwamm hergestellt ist, der eine Struktur hat, in welcher Poren gleichmäßig in einer dreidimensionalen Richtung verteilt sind und miteinander kontinuierlich sind, und die Grenzen zwischen benachbarten Poren von einem Harzfilm gebildet werden und in dem Harzfilm eine Öffnung ausgebildet ist, und bei welcher Urethanharz, gelöst in einem Lösungsmittel, in Koagulationsflüssigkeit koaguliert worden ist,
wobei der Feucht-Typ-Urethan-Schwamm einen maximalen Porendurchmesser, der nach dem Blasendruckverfahren, beschrieben in JIS K3832-1990, gemessen wird, von nicht weniger als 62 µm und nicht mehr als 113 µm hat.

2. Tupfer gemäß Anspruch 1, wobei
der sphärische Spitzenbereich (2) gebildet wird, indem zwei Stücke Schwamm (2A, 2B), die den Spitzenbereich des Griffbereichs (1) dazwischen angeordnet haben, an den Griffbereich (1) geschweißt werden, und
ein Basisendbereich des sphärischen Spitzenbereichs (2) eine Form hat, die sich mit zunehmendem Abstand von dem Spitzenbereich des Griffbereichs (1) im Durchmesser verkleinert.

3. Tupfer gemäß Anspruch 1 oder 2, wobei
der sphärische Spitzenbereich (2) eine im Wesentlichen zylindrische Form mit einer sphärisch geformten Spitze aufweist.

4. Tupfer gemäß Anspruch 3, wobei
der sphärische Spitzenbereich (2) außerdem einen schmalen zylindrischen Bereich (21) umfasst, der mit einer Basisendbereichsseite fortlaufend geformt ist.

## Revendications

1. Écouvillon comprenant :
une partie de poignée souple (1) ; et
une partie d'embout sphérique (2) prévue au niveau d'une partie d'embout de ladite partie de poignée (1) et composée d'une éponge en uréthane de type humide présentant une structure dans laquelle des pores sont répartis entre eux de manière uniforme dans une direction tridimensionnelle et continus, et les bordures entre des pores adjacents sont formées d'un film de résine et d'une ouverture formée dans ledit film de résine, et dans lequel de la résine d'uréthane dissoute dans un solvant a été coagulée dans un liquide de coagulation,
ladite éponge en uréthane de type humide ayant un diamètre de pores maximum, qui est mesuré par la méthode du point de bulle décrit dans la norme JIS K3832-1990, d'au moins 62 µm et d'au plus 113 µm.

2. Écouvillon selon la revendication 1, dans lequel
ladite partie d'embout sphérique (2) est formée en soudant deux morceaux d'éponge (2A, 2B) prenant en sandwich ladite partie d'embout de ladite partie de poignée (1) entre eux et ladite partie de poignée (1), et
une partie d'extrémité de base de ladite partie d'embout sphérique (2) a une forme dont le diamètre diminue en augmentant la distance à partir de ladite partie d'embout de ladite partie de poignée (1).

3. Écouvillon selon la revendication 1 ou 2, dans lequel
ladite partie d'embout sphérique (2) a une forme sensiblement cylindrique avec un embout de forme sphérique.

4. Écouvillon selon la revendication 3, dans lequel
ladite partie d'embout sphérique (2) inclut en outre une partie cylindrique mince (21) formée en continu avec un côté de partie d'extrémité de base.
